# EUROPEAN PATENT SPECIFICATION

(11) **EP 3 233 876 B1**
(45) Date of publication and mention of the grant of the patent: **20.02.2019**
(21) Application number: 15832914.4
(22) Date of filing: 18.12.2015
(51) Int. Cl.: C07H 1/08, C07H 3/02, C13K 1/02

(54) **PROCESS FOR THE PREPARATION OF A SACCHARIDE-CONTAINING SOLUTION FROM A TORREFIED CELLULOSIC BIOMASS**
VERFAHREN ZUR HERSTELLUNG EINER SACCHARIDENTHALTENDE LÖSUNG AUS GERÖSTETEM CELLULOSEHALTIGEM BIOMASS
PROCÉDÉ DE PRÉPARATION D'UNE SOLUTION CONTENANT UN SACCHARIDE À PARTIR DE BIOMASSE CELLULOSIQUE TORRÉFIÉE

(30) Priority: 18.12.2014 NL 2014005
(43) Date of publication of application: 25.10.2017
(73) Proprietor: Avantium Knowledge Centre B.V., 1014 BV Amsterdam (NL)
(72) Inventor: MCKAY, Benjamin, 1014 BV Amsterdam (NL); GRUTER, Gerardus Johannes Maria, 1014 BV Amsterdam (NL)
(74) Representative: EP&C
(86) International application number: PCT/NL2015/050881
(87) International publication number: WO 2016/099273

(56) References cited:
- EP-A1- 2 712 936
- US-A- 2 305 833
- US-A- 4 199 371
- US-A1- 2013 295 628
- Gerald Charles Atkinson Lindo: In: "THE CHEMISTRY AND STRUCTURE OF TORREFIED BIOMASS", 1 January 2010 (2010-01-01), Athens, Georgia, XP055187446, cited in the application pages 20,25,26 page 48

## Description

The present invention relates to a process for the preparation of a saccharide-containing solution from a cellulosic biomass feedstock.

Lignocellulose refers to plant dry matter biomass, so called lignocellulosic biomass. It is the most abundantly available raw material on the earth. It is composed of carbohydrate polymers, viz. cellulose and hemicellulose, and an aromatic polymer, viz. lignin. The carbohydrate polymers contain different sugar monomers; cellulose contains the hexose glucose only, while hemicellulose contains various sugars with six and five carbon atoms. They are tightly bound to lignin. Lignocellulosic biomass can be broadly classified into virgin biomass, waste biomass and energy crops, such as starch and sucrose-containing crops. Virgin biomass includes all naturally occurring terrestrial plants such as trees, bushes and grass. Waste biomass is produced as a low value byproduct of various industrial sectors such as agricultural, such as corn stover, sugarcane bagasse, straw, etc., waste paper, forestry, such as saw mill and paper mill discards. Energy crops are crops with high yield of lignocellulosic biomass produced to serve as a raw material for production of second generation biofuel. Examples include switch grass and miscanthus, also known as elephant grass.

One barrier to the production of carbohydrates, hereinafter also referred to as sugars, from biomass is that the sugars are trapped inside the lignocellulose. Lignocellulose has evolved to resist degradation and to confer hydrolytic stability and structural robustness to the cell walls of the plants. This robustness or "recalcitrance" is attributable to the crosslinking between the polysaccharides, i.e. cellulose and hemicellulose, and the lignin via ester and ether linkages. Ester linkages arise between oxidized sugars, the uronic acids, and phenols and phenylpropanols functionalities of the lignin. To extract the sugars, one must disconnect the celluloses from the lignin, and then hydrolyze the newly freed celluloses to break them down into soluble oligomers initially and simple monosaccharides eventually.

Another challenge to biomass valorization into biofuels or biochemicals is the high percentage of pentoses in the hemicellulose, such as xylose, or wood sugar. Unlike hexoses such as glucose or mannose, pentoses are difficult to ferment. For chemical conversions, relatively pure sugars are preferred rather than mixtures of C₅ and C₆ sugars. The problems presented by the lignin and hemicellulose fractions are the foci of much contemporary research. The different approaches used to liberate sugars from lignocellulosic biomass can be grouped as follows:
(1) Enzymatic hydrolysis (cf. US 2012/0104313 and US 2013/0078677).
(2) Dilute acid hydrolysis (cf. the Scholler or Madison processes as described in US 5879463 and DE 640775). EP 2712936 discloses a process for the manufacture of a sugar solution by mixing an acid, such as formic acid, acetic acid or a mixture thereof with a chloride salt to obtain an admixture, and hydrolyzing cellulosic biomass with this admixture.
(3) Hydrolysis using hot compressed or even supercritical water (cf. US 2013/239954).
(4) Concentrated acid hydrolysis at low temperatures, e.g. < 110 °C or even < 60 °C. Typical examples of acids include concentrated HCl, HBr, H₂SO₄ and HNO₃. Commercially this hydrolysis is applied in the Bergius-Rheinau process. The Bergius-Rheinau process using concentrated hydrochloric acid has been described in e.g. DE 362230 and US 4199371. A variation of the Hokkaido process that employs concentrated sulfuric acid has been described in US 5188673. A two-stage hydrolysis process of cellulose with concentrated hydrochloric acid has been described in US 2305833.

For many further applications, relatively pure glucose is the desired product. The above-mentioned methods have difficulty producing separated streams of sugars from hemicellulose, i.e. a mix of C₅ and C₆ sugars, and cellulose sugars, i.e. glucose. In some cases, such as with concentrated H₂SO₄ or with hot compressed or supercritical water, or with steam explosion as pretreatment, it has been either impossible or expensive to obtain relatively pure glucose by hydrolysis of lignocellulosic biomass.

In an attempt to produce bioethanol, a study was reported wherein biomass was pretreated by torrefaction or steam explosion and subsequently subjected to enzymatic hydrolysis and fermentation to obtain bioethanol (cf. D. Chiaramonti et al., Biomass Conv. Bioref., 1(1) (2011) 9-15). Torrefaction was studied in view of the expected decrease in moisture content and increase in grindability of the torrefied biomass. It was found that 1 kg of olive pruning chips after steam explosion result in 129 g ethanol, whereas the yield of ethanol was 16 g after grinding the chips without any other pre-treatment and just 14 g after torrefaction and grinding of the olive chips. It was concluded that torrefaction is considerably less energy efficient than steam explosion due to lower efficiencies in hydrolysis and fermentation.

In a thesis by G.C. Atkinson Lindo, "The chemistry and structure of torrefied biomass", Athens, Georgia, USA, 2010, it is described how torrefied wood samples are analyzed for their composition. Thereto, the samples are ground and digested with concentrated 72%wt sulfuric acid at room temperature for 1 hour. Subsequently, the samples were diluted to 4% acid, and a secondary hydrolysis was conducted for 11 hour at 121 °C. The samples of various torrefied materials were thus analyzed. The analyses showed that at the strongest torrefaction temperature applied, i.e. at 275 °C and for 3 hours, the yield of glucose was about 40.0%, the yield of xylose was 1.1% and 41.7% was undigested. The high proportion of undigested torrefied material shows that sulfuric acid has not hydrolyzed the ground particles satisfactorily.

The present invention has the objective to generate glucose in satisfactory yield and purity from biomass. The drawbacks of previous methods attempting to achieve this are overcome by using torrefied biomass material as starting material for the hydrolysis at conditions that are similar to those in the Bergius Rheinau process. Accordingly, the present invention provides a process for the preparation of a saccharide-containing solution from a cellulosic biomass feedstock, wherein the cellulosic biomass feedstock comprises torrefied biomass material, and wherein the torrefied biomass material is hydrolyzed in an aqueous reactant by contacting it with the aqueous reactant that comprises hydrochloric acid with a concentration of 35 to 45 %wt, based on the weight of the combination of hydrochloric acid and water, to yield a saccharide-containing solution.

Torrefaction of biomass is a known process, and may be described as a mild pyrolysis process. Whereas pyrolysis tends to be carried out at temperatures of at least 400 °C, torrefaction is conducted at temperatures in the range of 200 to 350 °C. The biomass torrefaction process takes place in an atmosphere of low oxygen content or in the substantial absence of oxygen. The pressure may suitably be atmospheric, although higher pressures may be applied. During torrefaction the water content of the biomass is greatly reduced. Further, volatile organic compounds are vaporized. In addition, the solid material of the biomass is also altered. In particular hemicellulose decomposes and yields volatile components and carbonaceous solids. The composition of lignin and cellulose in the torrefied biomass material is mostly maintained. The final product of torrefaction is a solid black material that comprises mainly lignin and cellulose. When the torrefied product is subjected to hydrolysis under the reaction conditions described above only the cellulose is hydrolyzed, yielding a solution containing glucose as monosaccharide and optionally glucose oligomers. Lignin may be removed and be used as fuel. Further, warm lignin in the torrefied product may act as binder when the torrefied product is pelletized, resulting in pellets with a higher bulk density than the original lignocellulosic biomass or the lignocellulosic biomass pellet, and in a mechanical strength of the pellet that is high. Therefore storage and transport of such pellets is easy. In addition, the torrefied biomass material is hydrophobic, which further facilitates its transport and storage significantly.

Torrefaction can be carried out in a variety of equipment types that are known in the art. One type of equipment is the rotating drum technology. In this technology the biomass is rotated in an inclined rotating drum, whilst being contacted with superheated steam or flue gas. A second type is a screw type reactor wherein the biomass is transported by means of an auger screw through the reactor. The reactor is usually heated indirectly using a heating medium inside a hollow wall of the reactor and/or a hollow screw. Direct heating is also possible, though. A third type is the so-called multiple hearth furnace. In this reactor the biomass is transported from one layer to another, wherein the layers have different temperature, gradually increasing from about 200 °C to the maximum torrefaction temperature, e.g. 350 °C. In a so-called torbed reactor a heating gas is passed at an angle through a bed of biomass resulting in a toroidal swirl. This reactor has been disclosed in US 2013/0104450. This reactor is stated to be excellently suited for cracking the hemicelluloses and leaving the lignin intact. A series of these reactors is used in the process of WO 2012/102617. The latter application shows how hemicellulose undergoes degassing and carbonization at a temperature in the range of 200 - 280 °C, whereas cellulose is more stable and does not start to degrade until a temperature of about 250 °C has been reached. The degradation of lignin proceeds very slowly.

Torrefaction may also be carried out in a moving bed reactor, a fluidized bed reactor, a belt dryer or a microwave reactor.

The gases that escape from the biomass during torrefaction can suitably be used for combustion and for the provision of heat that is necessary for the performance of the torrefaction reaction. An example thereof is described in US 8203024.

The cellulosic biomass feedstock that is used in the process of the present invention can originate from any biomass material. However, preferably, the biomass feedstock comprises torrefied lignocellulosic biomass material. The lignocellulosic material can originate from a variety of sources. Suitable examples thereof are wood, stover, grasses, bark, other leaves, other stalks, empty fruit bunches and combinations thereof. Waste paper is also a suitable feedstock. Wood is the preferred feedstock. The wood may originate from all types of trees, including spruce, willow, oak, birch, poplar, eucalyptus and other trees. The lignocellulosic material may be subjected to torrefaction in a variety of forms, including chips, pellets, powder, crushed particles, milled particles or ground particles. The thus torrefied lignocellulosic material results in cellulosic biomass feedstock. When the cellulosic biomass feedstock comprises wood the feedstock may be in the form of wood powder, wood chips, wood pellets, wood briquettes, wood chunks and combinations thereof. Preferably, the cellulosic biomass feedstock comprises more than 50%wt of torrefied biomass material, more preferably, more than 75 %wt, and most preferably consists for substantially 100%wt of torrefied biomass material, based on the cellulosic biomass material. The skilled person will realize that the cellulosic biomass material may comprise some moisture and/or some volatile compounds, at least some of which may have been formed during the torrefaction. The content of the moisture and volatile compounds is suitably below 50%wt, preferably below 25%wt and more preferably below 5%wt of the cellulosic biomass material. Most suitably the cellulosic biomass material is substantially moisture- and volatile compound-free.

As indicated above, the cellulosic biomass feedstock may be present in the form of pellets or chips. The pellet form provides a relatively high bulk density and is therefore advantageous for transportation purposes. For the hydrolysis it may be advantageous to provide a large surface area. This would point to the use of powder or ground particles. However, when the hydrolysis is carried out in practice the pressure drop over the bed of torrefied biomass and the compaction of such a bed renders the use of powder or ground particles unfeasible. One advantage of the present invention resides in the better penetrability of hydrochloric acid into chips or pellets of torrefied biomass compared to e.g. sulfuric acid, thereby improving the hydrolysis. The shape of the pellets of the chips or pellets may, e.g., be spherical or cylindrical. The length of the chip may suitably be in the range of 0.2 to 25 cm, preferably from 0.5 to 10 cm. The breadth may vary in the range of 0.1 to 5 cm. It is evident that these measures reflect the average length and minimum breadth. By length is meant the longest dimension and by breadth is meant the maximum dimension perpendicular to the length.

The torrefied biomass material has preferably been obtained by subjecting a lignocellulosic biomass starting material to a torrefaction method, including a treatment of the lignocellulosic biomass starting material to a temperature in the range of 200 to 350 °C in the substantial absence of oxygen. The torrefaction method has suitably been conducted for a period of 0.25 to 4 hr.

The cellulosic biomass feedstock usually comprises cellulose and lignin, and may optionally still comprise some hemicellulose. Suitably, the cellulosic biomass feedstock contains at most 5 %wt of hemicellulose, based on the weight of the biomass feedstock. The content of cellulose in the biomass feedstock may be as high as 75%wt, and is usually at least 25 %wt. The remainder comprises lignin, carbonaceous solids and ash. In addition, moisture and/or volatile compounds may also be present, as shown above.

Ash may be considered the inorganic residue that remains after combustion. It generally comprises inorganic salts and metal oxides. It may originate from the biomass itself or be introduced during any pre-treatment of the biomass. In this context it is especially advantageous to conduct the torrefaction as a wet torrefaction (developed by ECN, The Netherlands, under trade name Torwash) which allows for the combined torrefaction and washing of the biomass. The wet torrefaction, which is similar to a hydrothermal treatment, torrefies the biomass and, in addition, removes salts and minerals from the biomass. Thereby the quality of the resulting torrefied biomass material is further improved since it hardly contains any inorganic ash.

The hydrolysis of cellulose is known. US 2305833 describes the hydrolysis of cellulose-containing material using a strong hydrochloric acid solution. The process according to US 2305833 is directed to an improvement over methods that are known thus far, wherein the improvement resides in that the cellulose-containing material is not only contacted with a very strongly concentrated hydrochloric solution, but that the hydrolysis is achieved in two stages, wherein in the first stage the cellulose is contacted with a hydrochloric acid solution with a very high concentration, viz. such that the density is at least 1.16, and the resulting mixture is subsequently contacted with a less concentrated solution of hydrochloric acid in a second stage. Further developments in the hydrolysis of cellulose include the use of solid acids as described in US 8871739. In the process according to the latter patent cellulose-containing biomass is agitated in the presence of a solid acid. Solid acids include clays such as kaolinite, halloysite, attapulgite, montmorilloniteillite, nacrite and anauxite. Other suitable solid acids are aluminosilicates, such as zeolites. Super acids are a third group of solid acids. Examples thereof are alumina, zirconia or titania treated with sulfuric acid. According to another process according to US 8546560 carbonic acid is produced in situ by supercritical CO₂ and sub-critical, near-critical or supercritical water which is used to hydrolyze cellulose. None of these known processes discloses that torrefied biomass material would be an excellent feedstock for the hydrolysis of biomass.

The aqueous reactant is usually present in excess. Typically, the weight of the aqueous reactant may be from 1 to 1,000 times the amount of torrefied biomass material.

The torrefied biomass material is hydrolyzed in an aqueous reactant. As is known from the prior art, strong acids catalyze the hydrolysis of cellulose bonds at low temperatures, e.g. of at most 100 °C. Accordingly, the process of the present invention is carried out in an aqueous reactant that comprises hydrochloric acid. The aqueous reactant may also comprise other acids, e.g. organic acids. Therefore, the aqueous reactant may suitably comprise an organic acid, selected from the group consisting of formic acid, acetic acid, propionic acid, butyric acid, methane sulfonic acid, toluene sulfonic acid, trifluoroacetic acid and combinations thereof.

The concentration of the hydrochloric acid in the acid aqueous reactant that is employed in the process of the present invention is relatively high. In the process according to the present invention, like in the Bergius-Rheinau process, the concentration of hydrochloric acid may be as high as 35 to 45 %wt. Preferably, the hydrochloric acid concentration is 38 to 43 %wt, based on the weight of the combination of hydrochloric acid and water.

The hydrolysis of the torrefied biomass material may be carried out in one step. However, it may be advantageous to conduct the hydrolysis in at least two steps, wherein the second and optional subsequent steps are carried out in a less acidic reactant. Although the actual concentration of the acid employed may be reduced compared with the first step wherein a high concentration of the acid is used, the acid concentration is also in the second and optional subsequent steps such that the pH value does not drop below 4.0, preferably below 3.0, more preferably below 2.0 and most preferably below 1.0.

The torrefied biomass material is hydrolyzed in an aqueous reactant. The aqueous reactant may contain one or more other diluents. Suitable diluents are water-soluble organic compounds, such as alcohols, aldehydes, ketones, carboxylic acids, sulfoxides and combinations thereof. Examples of such compounds are methanol, ethanol, propanol-1, propanol-2, the butanols, formaldehyde, acetaldehyde, acetone, dimethylsulfoxide and combinations thereof.

In the process according to the present invention the pressure and temperature may be selected from a wide range by the skilled person. The pressure is not critical. Most effective is a pressure in the range of 1 to 10 bar. Sub-atmospheric pressure may be used but is not particularly desired since as acid vapors may escape from the concentrated hydrochloric acid that is used in the aqueous reactant. Pressures above 10 bar do not add any advantage to the process, whereas the costs for the equipment increase. The temperature at which the torrefied biomass material is exposed to the aqueous reactant may be selected from a range of temperatures. Suitably, the temperature is in the range of 0 to 110 °C, preferably from 10 to 80 °C, most preferably from 10 to 40 °C. When more than one step with different acidities is used, the temperature in the first step, wherein concentrated acid is used, is preferably within this range of 0 to 110 °C. The temperature of the second and subsequent steps is preferably in the range of 40 to 130 °C, preferably from 60 to 120 °C, and most preferably from 80 to 110 °C. If more than one step is employed it is advantageous to conduct the first step at the higher acid concentration and at a lower temperature, e.g. from 0 to 70 °C, and to carry out the second and optional further steps at a lower acid concentration and at a temperature in the range of 40 to 110 °C, wherein the temperature in the second and subsequent steps is higher than that in the first step. In the process of the present invention wherein hydrochloric acid is used the optimal temperature range is suitably from 10 to 45 °C for the entire single step hydrolysis or the first step of a multi-step hydrolysis.

The process according to the invention can be prolonged until the desired level of cellulose conversion has been obtained. Typically a quantitative conversion is aimed at. The residence times in the process may be similar to those applied in the prior art Bergius Rheinau process. It is known that the hydrolysis starts very quickly. Therefore, the hydrolysis of the torrefied biomass starting material in the presence of a concentrated hydrochloric acid solution is suitably continued for a period in the range of 0.1 to 24 hrs, preferably 1 to 20 hrs, more preferably from 5 to 16 hrs.

The resulting products of the hydrolysis of the torrefied biomass material tend to be solid materials comprising lignin and other solid carbon-containing compounds and an aqueous solution comprising saccharides. The solid material is suitably separated from the hydrolyzed torrefied biomass material, yielding a product comprising the saccharide-containing solution. The product may be separated by means of filtration, sedimentation, flotation, centrifugation or combinations thereof. Such may be the case when the hydrolysis is carried out in a stirred tank reactor, such as a stirred batch reactor, semi-batch reactor or a continuous stirred tank reactor (CSTR). In a preferred embodiment of the present process the hydrolysis is carried out similar to the Bergius-Rheinau process; the cellulosic biomass feedstock is placed in a fixed bed reactor as a bed of solid material, and an aqueous reactant is percolated from one end over the bed of solid material. The saccharide-containing aqueous solution is taken off at the other end of the fixed bed. Usually a series of fixed-bed reactors are provided, wherein the aqueous reactant is passed from one fixed-bed to another, wherein it is contacted with the biomass feedstock in a counter current manner. This method is particularly suitable for processes wherein concentrated hydrochloric is used as aqueous reactant. The aqueous saccharide-containing solution may comprise glucose as monosaccharide and glucose oligosaccharides, consisting of water-soluble cellodextrins, such as cellobiose or cellotriose.The aqueous saccharide-containing solution further comprises one or more acids.

The present process enables the skilled person for the first time to prepare a hydrolysate composition of torrefied biomass material that consists of an acid aqueous solution comprising saccharides wherein the amount of glucose and glucose oligomers is at least 90%wt, based on the carbohydrates in the hydrolysate, and wherein the acid in the aqueous solution comprises hydrochloric acid. In the hydrolysate of torrefied lignocellulosic biomass material that consists of an acid aqueous solution comprising mono- and oligosaccharides of mainly glucose, the weight ratio of glucose to pentose is suitably at least 90. The glucose and glucose oligomer content in such aqueous acid solutions is suitably at least 6%wt, preferably at least 10%wt, more preferably at least 30%wt based on the solution. The pH of the solution is suitably at most 1.0. Since the acid aqueous solution contains such a high glucose and glucose oligomer content and very little other sugars, it can advantageously be used for certain subsequent reactions. For example, glucose can be converted into hydroxymethylfurfural or levulinic acid in accordance with processes disclosed in US 8642791 or US 5892107, optionally after isomerization of glucose to fructose. These conversions take place in the presence of an acid catalyst. The hydrolysate obtained in the process according to the present invention suitably already contains an acid, so that the hydrolysate can be used directly in the desired conversion. Glucose obtained in the liquid product can be used for the preparation of a variety of other biobased chemicals as well. Examples include lactic acid, succinic acid, sorbitol, isosorbide, fructose, butane diol, butadiene and alkylene glycols, such as ethylene glycol, propylene glycol or butulene glycol. The lignin obtained after separation can be used as fuel.

The invention is further illustrated by means of the following example.

### EXAM PLES

### COMPARATIVE EXAMPLE

Two types of torrefied wood pellets were used in this Comparative Example as Biomass Feedstock 1 and 2. Biomass Feedstock 1 consisted of torrefied poplar wood pellets. The pellets had a cylindrical shape and had a length of about 0.8 cm and a breadth of about 0.5 cm. Biomass Feedstock 2 comprised the torrefied product of a different wood. Portions of the Biomass Feedstocks were crushed to yield wood powder. The pellets and the dust samples were used in five experiments. The cellulose content in the Biomass Feedstocks was assessed to be more than about 55%wt, the hemicelluloses content was about 5%wt and the lignin content was less than about 40 %wt, based on the total pellets. By thorough drying Biomass Feedstocks lost about 15 %wt of moisture and volatile components ("Dried Feedstocks").

In Experiments 1 and 3 the wood pellets of Biomass Feedstock 1 and 2 were mixed with a sulfuric acid solution having a sulfuric acid concentration of 70%wt. The weight ratio of the solution to the Feedstock was 10:1. This boiled down to a weight ratio of acid to cellulose of 11.5 in both experiments. The mixtures obtained were gently stirred at room temperature for a period of 4 hrs to achieve hydrolysis of cellulose.

The thus hydrolyzed mixtures were filtered to yield clear solutions. Portions of these solutions were diluted with water to reach a sulfuric acid concentration of 30%wt. The solutions contained oligosaccharides, e.g. cellobiose, and monosaccharides. In order to complete the hydrolysis the diluted portions were placed in an oven at 80 for 4 hrs. To separate the sugars from any solids formed, the resulting mixtures were centrifuged to yield a clear liquid. The products in the centrifuged liquids were analyzed. The results are shown in the Table below. The percentages of the products are based on the weight of the Dried Feedstocks to be hydrolyzed.

In Experiments 2 and 4 powder particles from the respective Feedstocks were subjected to the identical treatments as in Experiments 1 and 3. The results of these Experiments are also shown in the Table.

In Experiment 5 wood pellets of Biomass Feedstock 2 were mixed with a 70%wt solution of sulfuric acid in an aqueous mixture of 10%wt ethanol and 90%wt water. The weight ratio of the mixture to the wood pellets was 10:1, boiling down to an acid to cellulose weight ratio of 11.5. The mixture was subjected to an identical treatment as described for Experiments 1 and 3. The results of Experiments 5 are shown in the Table 1 below.

**Table 1**

| Exp. No. | Biomass Feedstock | Pellets/Dust | Product, %wt | | | |
|---|---|---|---|---|---|---|
| | | | Cellobiose | Glucose | Mannose | C₅-sugars |
| 1 | 1 | Pellets | 0.0 | 4.5 | 0.4 | 0.0 |
| 2 | 1 | Powder | 0.7 | 43.5 | 3.2 | 0.0 |
| 3 | 2 | Pellets | 0.0 | 6.1 | 1.1 | 0.1 |
| 4 | 2 | Powder | 0.0 | 33.2 | 6.0 | 0.4 |
| 5 | 2 | Pellets | 0.0 | 7.4 | 1.2 | 0.0 |

The results show that the torrefied biomass material yields hydrolysate with an excess of hexoses, in particular glucose, compared to any pentose, i.e. arabinose or xylose. The results further show that finely divided powder hydrolyzed with sulfuric acid yields a higher concentration of glucose than wood pellets.

### EXAM PLE

Torrefied poplar wood pellets from Biomass Feedstock 1 were used in this Example. The pellets were of a similar size as those in the above-described Comparative Example.

In the Example the wood pellets were mixed with a hydrochloric acid solution having a hydrochloric acid concentration of 41%wt. The weight ratio of the solution to the wood pellets was 5:1. This boiled down to a weight ratio of acid to cellulose of 3.75 in this example. The mixture obtained was gently stirred at room temperature to achieve hydrolysis of cellulose. A first portion was stirred for a period of 4 hrs. The thus hydrolyzed portion was filtered to remove solids and the resulting filtrate was diluted with water to reach a hydrochloric acid concentration of 13%wt. In order to complete the hydrolysis the diluted filtrate was placed in an oven at 80 °C for 20 hrs.

A second portion was hydrolyzed whilst stirring at room temperature for 21 hrs. The resulting mixture was filtered to remove solids and the resulting filtrate was diluted with water to a hydrochloric acid concentration of 4.7 %wt. To complete the hydrolysis the diluted filtrate was placed in an oven at 80 °C for 20 hrs.

Both portions were subjected to solids/liquid separation to ensure that clear solutions were obtained. The products in the clear solutions were analyzed. The results are shown in Table 2 below. The percentages of the products are based on the weight of the Dried Feedstock.

**Table 2**

| Exp. No. | Hydrolysis duration, hr | Product, %wt | | | |
|---|---|---|---|---|---|
| | | Cellobiose | Glucose | Mannose | C₅-sugars |
| 6 | 4 | 0 | 17.8 | 1.9 | 0.9 |
| 7 | 21 | 0 | 73.4 | 6.1 | 3.1 |

Comparison between Experiment Nos. 1 and 6 shows that the use of hydrochloric acid yields a significantly higher yield of monosaccharides than the use of sulfuric acid when pellets are used. Experiment No. 7 further shows that when the pellets are subjected to prolonged hydrolysis, the yield of monosaccharides is further enhanced to higher levels than the hydrolysis of powder by sulfuric acid. The skilled person may thus select the duration of the first step of the hydrolysis depending on the desired yield of monoaccharides.

## Claims

1. Process for the preparation of a saccharide-containing solution from a cellulosic biomass feedstock, wherein the cellulosic biomass feedstock comprises torrefied biomass material, and wherein the torrefied biomass material is hydrolyzed in an aqueous reactant by contacting it with the aqueous reactant that comprises hydrochloric acid with a concentration of 35 to 45 %wt, based on the weight of the combination of hydrochloric acid and water, to yield a saccharide-containing solution.

2. Process according to claim 1, wherein the cellulosic biomass feedstock comprises torrefied lignocellulosic biomass material.

3. Process according to claim 2, wherein the lignocellulosic biomass material has been selected from the group consisting of wood, stover, grasses, other leaves, other stalks, empty fruit bunches, waste paper and combinations thereof.

4. Process according to any one of claims 1 to 3, wherein the torrefied biomass material is in the form of chips or pellets.

5. Process according to any one of claims 1 to 4, wherein torrefied biomass material has been obtained by subjecting a lignocellulosic biomass starting material to a torrefaction method, including a treatment of the lignocellulosic biomass starting material to a temperature in the range of 200 to 350 °C in the substantial absence of oxygen.

6. Process according to claim 5, wherein the torrefaction method was conducted for a period of 0.25 to 4 hr.

7. Process according to any one of claims 1 to 6, wherein the cellulosic biomass feedstock contains at most 5 %wt of hemicellulose, based on the weight of the cellulosic biomass feedstock.

8. Process according to any one of claims 1 to 7, wherein the torrefied biomass material is hydrolyzed at a temperature of 0 to 110 °C.

9. Process according to any one of claims 1 to 8, wherein a product comprising the saccharide-containing solution is separated from the hydrolyzed torrefied biomass.

10. Process according to any one of claims 1 to 9, wherein the cellulosic biomass feedstock is placed in a fixed bed reactor as a bed of solid material, and an aqueous reactant is percolated from one end over the bed of solid material wherein the saccharide-containing solution is taken off at the other end of the fixed bed.

11. Hydrolysate composition of torrefied biomass material that consists of an acid aqueous solution comprising monosaccharides,
wherein the amount of glucose and glucose oligomers is at least 90%wt, based on the carbohydrates in the hydrolysate; and
wherein the acid in the aqueous solution comprises hydrochloric acid.

12. Hydrolysate composition according to claim 11, wherein the glucose content in the aqueous acid solution is at least 6%wt, based on the solution.

13. Hydrolysate composition according to any one of claims 11 to 12 , wherein the acid aqueous solution has a pH of at most 1.0.

## Patentansprüche

1. Verfahren zur Herstellung einer saccharidhaltigen Lösung aus einem cellulosischen Biomasse-Rohstoff, wobei der cellulosische Biomasse-Rohstoff geröstetes Biomasse-Material umfasst, und wobei das geröstete Biomasse-Material in einem wässrigen Reaktanten hydrolisiert ist, indem es mit dem wässrigen Reaktanten in Kontakt gebracht wird, der Salzsäure mit einer Konzentration von 35 bis 45 Gew.-% basierend auf dem Gewicht der Kombination von Salzsäure und Wasser umfasst, um eine saccharidhaltige Lösung zu erzeugen.

2. Verfahren nach Anspruch 1, wobei der cellulosische Biomasse-Rohstoff geröstetes lignocellulosisches Biomasse-Material umfasst.

3. Verfahren nach Anspruch 2, wobei das lignocellulosische Biomasse-Material ausgewählt wurde aus der Gruppe bestehend aus Holz, Stroh, Gräser, andere Blätter, andere Stiele, leere Fruchtbüschel, Altpapier und Kombinationen davon.

4. Verfahren nach einem der Ansprüche 1 bis 3, wobei das geröstete Biomasse-Material in der Form von Holzspänen oder Pellets ist.

5. Verfahren nach einem der Ansprüche 1 bis 4, wobei geröstetes Biomasse-Material dadurch erhalten wurde, dass ein lignocellulosisches Biomasse-Anfangsmaterial einem Röstverfahren unterzogen wurde, das eine Behandlung des lignocellulosischen Biomasse-Ausgangsmaterials auf eine Temperatur im Bereich von 200 bis 350 °C bei wesentlicher Abwesenheit von Sauerstoff einschließt.

6. Verfahren nach Anspruch 5, wobei das Röstverfahren für einen Zeitraum von 0,25 bis 4 Std. ausgeführt wurde.

7. Verfahren nach einem der Ansprüche 1 bis 6, wobei der cellulosische Biomasse-Rohstoff höchstens 5 Gew.-% Hemicellulose basierend auf dem Gewicht des cellulosischen Biomasse-Rohstoffs enthält.

8. Verfahren nach einem der Ansprüche 1 bis 7, wobei das geröstete Biomasse-Material bei einer Temperatur von 0 bis 110 °C hydrolisiert wird.

9. Verfahren nach einem der Ansprüche 1 bis 8, wobei ein Produkt, das die saccharidhaltige Lösung umfasst, von der hydrolisierten gerösteten Biomasse getrennt wird.

10. Verfahren nach einem der Ansprüche 1 bis 9, wobei der cellulosische Biomasse-Rohstoff in einem Festbettreaktor als ein Bett aus festem Material platziert wird, und ein wässriger Reaktant von einem Ende über das Bett aus festem Material perkoliert wird, wobei die saccharidhaltige Lösung am anderen Ende des Festbetts entnommen wird.

11. Hydrolysatzusammensetzung von geröstetem Biomasse-Material, das aus einer sauren wässrigen Lösung besteht, die Monosaccharide umfasst,
wobei die Menge an Glucose und Glucose-Oligomeren mindestens 90 Gew.-% basierend auf den Kohlenhydraten im Hydrolysat ist; und
wobei die Säure in der wässrigen Lösung Salzsäure umfasst.

12. Hydrolysatzusammensetzung nach Anspruch 11, wobei der Glucosegehalt in der wässrigen sauren Lösung mindestens 6 Gew.-% basierend auf der Lösung ist.

13. Hydrolysatzusammensetzung nach einem der Ansprüche 11 bis 12, wobei die saure wässrige Lösung einen pH-Wert von höchstens 1,0 aufweist.

## Revendications

1. Procédé de préparation d'une solution contenant un saccharide à partir d'une charge d'alimentation de biomasse cellulosique, dans lequel la matière première de biomasse cellulosique comprend un matériau de biomasse torréfié, et dans lequel le matériau de biomasse torréfié est hydrolysé dans un réactif aqueux en le mettant en contact avec le réactif aqueux, qui comprend de l'acide chlorhydrique à une concentration allant de 35 à 45% en poids, sur la base du poids de la combinaison d'acide chlorhydrique et d'eau, pour donner une solution contenant du saccharide.

2. Procédé selon la revendication 1, dans lequel la charge d'alimentation de biomasse cellulosique comprend un matériau de biomasse lignocellulosique torréfié.

3. Procédé selon la revendication 2, dans lequel la charge d'alimentation de biomasse lignocellulosique a été choisie dans le groupe consistant en bois, fourrage, herbes, autres feuilles, autres tiges, grappes de fruits vides, vieux papiers et des combinaisons de ceux-ci.

4. Procédé selon l'une quelconque des revendications 1 à 3, dans lequel le matériau de biomasse torréfié est sous la forme de copeaux ou de pastilles.

5. Procédé selon l'une quelconque des revendications 1 à 4, dans lequel le matériau de biomasse torréfié a été obtenu en soumettant un matériau de départ de biomasse lignocellulosique à un procédé de torréfaction, comprenant un traitement du matériau de départ de biomasse lignocellulosique à une température de la gamme allant de 200 à 350°C en l'absence substantielle d'oxygène.

6. Procédé selon la revendication 5, dans lequel le procédé de torréfaction a été conduit pendant une période de 0,25 à 4 heures.

7. Procédé selon l'une quelconque des revendications 1 à 6, dans lequel la charge d'alimentation de biomasse cellulosique contient au plus 5% en poids d'hémicellulose, sur la base du poids de la charge d'alimentation de biomasse cellulosique.

8. Procédé selon l'une quelconque des revendications 1 à 7, dans lequel le matériau de biomasse torréfié est hydrolysé à une température de 0 à 110°C.

9. Procédé selon l'une quelconque des revendications 1 à 8, dans lequel un produit comprenant la solution contenant du saccharide est séparé de la biomasse torréfiée hydrolysée.

10. Procédé selon l'une quelconque des revendications 1 à 9, dans lequel la charge d'alimentation de biomasse cellulosique est placée dans un réacteur à lit fixe sous la forme d'un lit de matériau solide, et un réactif aqueux est percolé à partir d'une extrémité dans le lit de matériau solide dans lequel la solution contenant du saccharide est retirée à l'autre extrémité du lit fixe.

11. Composé d'hydrolysat de matériau de biomasse torréfié qui consiste en une solution aqueuse acide comprenant des monosaccharides,
dans lequel la quantité de glucose et d'oligomères de glucose est d'au moins 90% en poids, sur la base des hydrates de carbone de l'hydrolysat ; et
dans lequel l'acide dans la solution aqueuse comprend de l'acide chlorhydrique.

12. Composé d'hydrolysat selon la revendication 11, dans lequel la teneur en glucose de la solution aqueuse acide est d'au moins 6% en poids, sur la base de la solution.

13. Composé d'hydrolysat selon l'une quelconque des revendications 11 à 12, dans lequel la solution aqueuse acide a un pH d'au plus 1,0.
